# FASCICULE DE BREVET EUROPEEN

(11) **EP 3 074 342 B1**
(45) Date de publication et mention de la délivrance du brevet: **17.01.2018**
(21) Numéro de dépôt: 14809480.8
(22) Date de dépôt: 10.11.2014
(51) Int. Cl.: C01B 7/07, B01D 53/68, C07C 17/20, B01D 53/04, B01D 53/78, B01D 53/86, C07C 17/25, C07C 17/395

(54) **PROCEDE DE PURIFICATION D'ACIDE CHLORHYDRIQUE**
SALZSÄURE-REINIGUNGSVERFAHREN
HYDROCHLORIC ACID PURIFICATION PROCESS

(30) Priorité: 28.11.2013 FR 1361736
(43) Date de publication de la demande: 05.10.2016
(73) Titulaire: Arkema France, 92700 Colombes (FR)
(72) Inventeur: COLLIER, Bertrand, F-69230 Saint-genis-Laval (FR); DEUR-BERT, Dominique, F-69390 Charly (FR); LACAMBRA, Joaquin, F-69390 Vernaison (FR); PIGAMO, Anne, F-69340 Francheville (FR)
(74) Mandataire: Leca, François Michel
(86) Numéro de dépôt international: PCT/FR2014/052868
(87) Numéro de publication internationale: WO 2015/079137

(56) Documents cités:
- WO-A1-02/099005
- WO-A2-2007/079431
- FR-A- 1 507 252
- FR-A1- 2 019 406
- US-A- 3 074 779
- US-A- 3 353 911
- US-A- 3 464 787
- US-A- 4 902 838

## Description

### DOMAINE DE L'INVENTION

La présente invention concerne un procédé de purification d'acide chlorhydrique, ainsi qu'une installation adaptée à la mise en oeuvre de ce procédé. L'invention peut être en particulier utilisée dans le cadre du traitement des effluents issus d'une réaction de fluoration catalytique.

### ARRIERE-PLAN TECHNIQUE

Il est connu de produire des composés fluorés tels que les hydrofluorocarbures par fluoration de composés chlorés tels que les hydrochlorocarbures notamment. Cette fluoration est généralement une fluoration catalytique utilisant l'acide fluorhydrique (HF) comme agent fluorant.

Au cours de ce type de réaction, de l'acide chlorhydrique (HCl) est coproduit. Il est connu de séparer le HCl des autre gaz produits par l'intermédiaire d'une colonne de distillation, puis d'absorber le HCl dans une colonne d'absorption adiabatique pour générer une solution d'HCl de type commercial.

Le document FR 1507252 décrit des étapes de traitement sur charbon actif, à haute température et en présence d'eau, et de lavage avec de l'acide chlorhydrique aqueux concentré.

Le document US 3,353,911 décrit la purification d'un mélange gazeux HF / HCl par mise en contact avec une solution absorbante, qui est une solution saturée d'acide borique.

Toutefois, les techniques connues de purification de l'acide chlorhydrique ne permettent pas, dans certains cas, d'atteindre la pureté en HCl requise.

Il existe donc un besoin de fournir un procédé amélioré de purification de l'acide chlorhydrique dans un flux gazeux.

### RESUME DE L'INVENTION

L'invention concerne en premier lieu un procédé de traitement d'un flux gazeux contenant de l'acide chlorhydrique, de l'acide fluorhydrique et des composés fluoro-oxygénés, dans lequel le flux gazeux est successivement soumis à :
- une étape d'hydrolyse des composés fluoro-oxygénés;
- une étape de lavage par une solution acide ;
- une étape d'adsorption d'impuretés par du charbon actif ;
- une étape d'absorption adiabatique ou isotherme de l'acide chlorhydrique dans une solution aqueuse, permettant de collecter une solution d'acide chlorhydrique.

Selon un mode de réalisation, l'étape d'hydrolyse catalytique est effectuée sur un lit de charbon actif.

Selon un mode de réalisation, la solution acide utilisée lors de l'étape de lavage est une solution d'acide chlorhydrique et de préférence provient de la solution d'acide chlorhydrique collectée à l'issue de l'étape d'absorption adiabatique ou isotherme.

Selon un mode de réalisation, le procédé comprend en outre :
- une étape de mise en contact de la solution d'acide chlorhydrique avec un gel de silice.

Selon un mode de réalisation, les composés fluoro-oxygénés comprennent du difluorure de carbonyle, du chlorofluorocarbonyle, du fluorure de trifluoroacétyle et/ou de l'acide trifluoroacétique ; et de préférence, le flux gazeux comprend au moins 50 mg/L, notamment au moins 100 mg/L, voire au moins 200 mg/L de fluorure de trifluoroacétyle et/ou acide trifluoroacétique.

Selon un mode de réalisation, de l'acide borique est ajouté à la solution acide utilisée pour l'étape de lavage.

Selon un mode de réalisation, le flux gazeux est un flux issu d'une réaction de fluoration catalytique d'au moins un composé chloré en au moins un composé fluoré, ledit flux étant de préférence collecté en sortie d'une distillation d'un flux de produits de la réaction de fluoration catalytique.

Selon un mode de réalisation, la réaction de fluoration catalytique est effectuée en présence d'oxygène.

Selon un mode de réalisation :
- le composé chloré est un chlorocarbure, un hydrochlorocarbure, un chlorofluorocarbure, un hydrochlorofluorocarbure, une chlorooléfine, une hydrochlorooléfine, une chlorofluorooléfine ou une hydrochlorofluorooléfine ; et le composé fluoré est un fluorocarbure, un hydrofluorocarbure, un chlorofluorocarbure, un hydrochlorofluorocarbure, une fluorooléfine, une hydrofluorooléfine, une chlorofluorooléfine ou une hydrochlorofluorooléfine ;
- de préférence le composé chloré est choisi parmi le 1,1,2-trichloroéthane, le 1,1,1,2,3-pentachloropropane, le 1,1,1,3,3-pentachloropropane, le 1,1,2,2,3-pentachloropropane, le 2,3-dichloro-1,1,1-trifluoropropane, le perchloroéthylène, le 1,2-dichloroéthylène, le 1,1,2,3-tétrachloropropène, le 2,3,3,3-tetrachloropropène, le 1,1,3,3-tétrachloropropène, le 1,3,3,3-tétrachloropropène, le 2-chloro-3,3,3-trifluoropropène, le 1-chloro-3,3,3-trifluoropropène et leurs mélanges ; et
- de préférence le composé fluoré est choisi parmi le pentafluoroéthane, le 1-chloro,2,2-difluoroéthane, le 1,3,3,3-tétrafluoropropène, le 2,3,3,3-tétrafluoropropène, le 2 chloro-3,3,3-trifluoropropène, le 1-chloro-3,3,3-trifluoropropène et leurs mélanges.

L'invention concerne également un procédé de préparation d'un composé fluoré comprenant :
- la fourniture d'un composé chloré et d'acide fluorhydrique ;
- la réaction catalytique du composé chloré avec l'acide fluorhydrique et la collecte d'un flux de produits bruts ;
- la séparation du flux de produits bruts, de préférence par distillation, permettant de récupérer d'une part un flux de composé fluoré, et d'autre part un flux gazeux contenant principalement de l'acide chlorhydrique, ainsi que des quantités minoritaires d'acide fluorhydrique et de composés fluoro-oxygénés ;
- le traitement du flux gazeux contenant de l'acide chlorhydrique, de l'acide fluorhydrique et des composés fluoro-oxygénés tel que décrit ci-dessus.

Selon un mode de réalisation, la réaction catalytique est mise en oeuvre en présence d'oxygène.

Selon un mode de réalisation :
- le composé chloré est un chlorocarbure, un hydrochlorocarbure, un chlorofluorocarbure, un hydrochlorofluorocarbure, une chlorooléfine, une hydrochlorooléfine, une chlorofluorooléfine ou une hydrochlorofluorooléfine ; et le composé fluoré est un fluorocarbure, un hydrofluorocarbure, un chlorofluorocarbure, un hydrochlorofluorocarbure, une fluorooléfine, une hydrofluorooléfine, une chlorofluorooléfine ou une hydrochlorofluorooléfine ;
- de préférence le composé chloré est choisi parmi le 1,1,2-trichloroéthane, le 1,1,1,2,3-pentachloropropane, le 1,1,1,3,3-pentachloropropane, le 1,1,2,2,3-pentachloropropane, le 2,3-dichloro-1,1,1-trifluoropropane, le perchloroéthylène, le 1,2-dichloroéthylène, le 1,1,2,3-tétrachloropropène, le 2,3,3,3-tetrachloropropène, le 1,1,3,3-tétrachloropropène, le 1,3,3,3-tétrachloropropène, le 2-chloro-3,3,3-trifluoropropène, le 1-chloro-3,3,3-trifluoropropène et leurs mélanges ; et
- de préférence le composé fluoré est choisi parmi le pentafluoroéthane, le 1-chloro,2,2-difluoroéthane, le 1,3,3,3-tétrafluoropropène, le 2,3,3,3-tétrafluoropropène, le 2 chloro-3,3,3-trifluoropropène, le 1-chloro-3,3,3-trifluoropropène et leurs mélanges.

L'invention concerne également une installation de traitement d'un flux gazeux contenant de l'acide chlorhydrique, de l'acide fluorhydrique et des composés fluoro-oxygénés, comprenant :
- une conduite d'amenée de flux gazeux contenant de l'acide chlorhydrique, de l'acide fluorhydrique et des composés fluoro-oxygénés, alimentant une unité d'hydrolyse catalytique ;
- une unité de lavage, alimentée d'une part par une conduite de collecte de flux gazeux hydrolysé issue de l'unité d'hydrolyse catalytique, et d'autre part par une conduite d'amenée de solution acide ;
- une unité d'adsorption d'impuretés, comprenant un lit de charbon actif, alimentée par une conduite de collecte de flux gazeux lavé issue de l'unité de lavage ;
- une unité d'absorption adiabatique ou isotherme alimentée d'une part par une conduite de collecte de flux purifié ou isotherme issue de l'unité d'adsorption et d'autre part par une conduite d'amenée de solution aqueuse ;
- une conduite de collecte de solution d'acide chlorhydrique en sortie de l'unité d'absorption adiabatique ou isotherme.

Selon un mode de réalisation, l'unité d'hydrolyse catalytique comprend un lit de charbon actif.

Selon un mode de réalisation, la conduite d'amenée de solution acide est issue directement ou non de la conduite de collecte de solution d'acide chlorhydrique.

Selon un mode de réalisation, l'installation comprend en outre :
- une unité d'adsorption complémentaire comprenant un gel de silice, alimentée par la conduite de collecte de solution d'acide chlorhydrique ; et
- une conduite de collecte de solution d'acide chlorhydrique purifiée issue de l'unité d'adsorption supplémentaire.

Selon un mode de réalisation, l'installation comprend un apport de solution d'acide borique à l'unité de lavage.

Selon un mode de réalisation, la conduite d'amenée de flux gazeux contenant de l'acide chlorhydrique, de l'acide fluorhydrique et des composés fluoro-oxygénés est issue d'une unité de distillation ; l'unité de distillation étant de préférence alimentée par une conduite de collecte de produits bruts en sortie d'un réacteur catalytique.

Selon un mode de réalisation, le réacteur catalytique est alimenté par une conduite d'amenée de composé chloré et une conduite d'amenée d'acide fluorhydrique, et la conduite de collecte de produits bruts transporte un flux comprenant un composé fluoré, et de préférence :
- le composé chloré est un chlorocarbure, un hydrochlorocarbure, un chlorofluorocarbure, un hydrochlorofluorocarbure, une chlorooléfine, une hydrochlorooléfine, une chlorofluorooléfine ou une hydrochlorofluorooléfine ; et le composé fluoré est un fluorocarbure, un hydrofluorocarbure, un chlorofluorocarbure, un hydrochlorofluorocarbure, une fluorooléfine, une hydrofluorooléfine, une chlorofluorooléfine ou une hydrochlorofluorooléfine ;
- plus particulièrement le composé chloré est choisi parmi le 1,1,2-trichloroéthane, le 1,1,1,2,3-pentachloropropane, le 1,1,1,3,3-pentachloropropane, le 1,1,2,2,3-pentachloropropane, le 2,3-dichloro-1,1,1-trifluoropropane, le perchloroéthylène, le 1,2-dichloroéthylène, le 1,1,2,3-tétrachloropropène, le 2,3,3,3-tetrachloropropène, le 1,1,3,3-tétrachloropropène, le 1,3,3,3-tétrachloropropène, le 2-chloro-3,3,3-trifluoropropène, le 1-chloro-3,3,3-trifluoropropène et leurs mélanges ; et
- plus particulièrement le composé fluoré est choisi parmi le pentafluoroéthane, le 1-chloro,2,2-difluoroéthane, le 1,3,3,3-tétrafluoropropène, le 2,3,3,3-tétrafluoropropène, le 2 chloro-3,3,3-trifluoropropène, le 1-chloro-3,3,3-trifluoropropène et leurs mélanges.

Selon un mode de réalisation, l'installation comprend un apport d'oxygène vers le réacteur catalytique.

La présente invention permet de surmonter les inconvénients de l'état de la technique. Elle fournit plus particulièrement un procédé amélioré de purification de l'acide chlorhydrique dans un flux gazeux.

Cela est accompli grâce à la mise en oeuvre de trois étapes de traitement successives du flux gazeux, à savoir une étape d'hydrolyse catalytique, une étape de lavage et une étape d'adsorption sur charbon actif, et ce avant l'étape d'absorption adiabatique ou isotherme générant une solution d'acide chlorhydrique concentrée.

L'invention repose sur l'identification par les présents inventeurs que l'HCl gazeux récupéré en tête de colonne de distillation peut être contaminé par une faible quantité d'HF libre (entraîné en raison d'azéotropes avec des composés fluorés légers) mais également par des composés fluoro-oxygénés, tels que le difluorure de carbonyle (COF₂), le chlorofluorure de carbonyle (COFCl) et le fluorure de trifluoroacétyle (CF₃COF).

Ces composés sont en particulier générés lorsque le flux gazeux à traiter est issu d'une réaction de fluoration mise en oeuvre en présence d'oxygène.

Ces composés sont fortement toxiques et sont hydrolysables. Ils sont donc susceptibles de libérer de l'HF notamment lors de l'étape d'absorption adiabatique ou isotherme dans l'eau, contaminant ainsi la solution de HCl obtenue avec du HF. En outre, le fluorure de trifluoroacétyle génère de l'acide trifluoroacétique (ou TFA) lors de son hydrolyse, composé qui est nocif.

L'invention permet de séparer le HCl du HF avec lequel il est mélangé, mais également de le séparer des composés fluoro-oxygénés mentionnés ci-dessus.

L'invention repose également sur l'identification par les présents inventeurs que l'HCl gazeux récupéré en tête de colonne de distillation peut être contaminé par des composés organiques légers. L'invention permet également d'éliminer ces composés organiques légers de façon satisfaisante lors du traitement.

### BREVE DESCRIPTION DES FIGURES

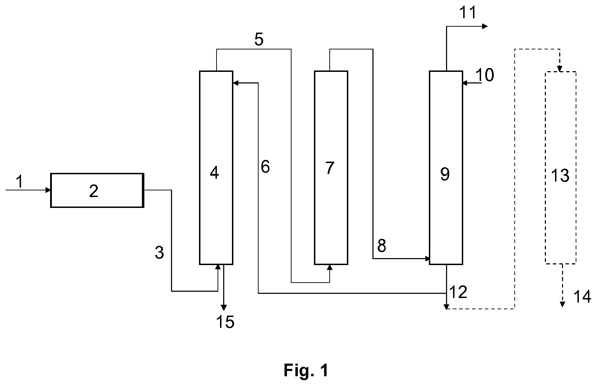

La **figure 1** représente de manière schématique un mode de réalisation d'une installation selon l'invention.

### DESCRIPTION DE MODES DE REALISATION DE L'INVENTION

L'invention est maintenant décrite plus en détail et de façon non limitative dans la description qui suit.

L'invention s'applique en particulier au traitement d'un flux gazeux issu d'une réaction de fluoration catalytique d'au moins un composé chloré en au moins un composé fluoré. Le flux gazeux traité selon l'invention peut être directement le flux gazeux de produits issu du réacteur, ou plus préférentiellement un flux issu d'une distillation (mise en oeuvre dans une colonne de distillation ou dans une série de plusieurs colonnes de distillation successives) à l'issue de la fluoration catalytique. Dans ce cas, le flux gazeux à traiter selon l'invention a préalablement été essentiellement séparé du composé fluoré et/ou du composé chloré non réagi et/ou des produits secondaires de la réaction.

Le flux gazeux à traiter selon l'invention contient de préférence majoritairement du HCl, avec des quantités minoritaires de contaminants tels que le HF et les composés fluoro-oxygénés susmentionnés (et notamment du fluorure de trifluoroacétyle).

Par composé chloré (qui représente le réactif principal de la réaction de fluoration catalytique), on entend un composé organique comprenant un ou plusieurs atomes de chlore, et par composé fluoré (qui représente le produit recherché de la réaction de fluoration catalytique), on entend un composé organique comprenant un ou plusieurs atomes de fluor.

Il est entendu que le composé chloré peut comprendre un ou plusieurs atomes de fluor, et que le composé fluoré peut comprendre un ou plusieurs atomes de chlore. De manière générale, le nombre d'atomes de chlore du composé fluoré est inférieur au nombre d'atomes de chlore du composé chloré ; et le nombre d'atomes de fluor du composé fluoré est supérieur au nombre d'atomes de fluor du composé chloré.

Le composé chloré peut être un alcane ou un alcène ayant éventuellement des substituants choisis parmi F, Cl, I et Br (de préférence parmi F et Cl), et comportant au moins un substituant Cl.

Le composé fluoré peut être un alcane ou un alcène ayant éventuellement des substituants choisis parmi F, Cl, I et Br (de préférence parmi F et Cl), et comportant au moins un substituant F.

Le composé chloré peut notamment être un alcane avec un ou plusieurs substituants chlore (hydrochlorocarbure ou chlorocarbure) ou un alcane avec un ou plusieurs substituants chlore et fluor (hydrochlorofluorocarbure ou chlorofluorocarbure) ou un alcène avec un ou plusieurs substituants chlore (chlorooléfine ou hydrochlorooléfine) ou un alcène avec un ou plusieurs substituants chlore et fluor (hydrochlorofluorooléfine ou chlorofluorooléfine).

Le composé fluoré peut notamment être un alcane avec un ou plusieurs substituants fluor (fluorocarbure ou hydrofluorocarbure) ou un alcane avec un ou plusieurs substituants chlore et fluor (hydrochlorofluorocarbure ou chlorofluorocarbure) ou un alcène avec un ou plusieurs substituants fluor (fluorooléfine ou hydrofluorooléfine) ou un alcène avec un ou plusieurs substituants chlore et fluor (hydrochlorofluorooléfine ou chlorofluorooléfine).

Le composé chloré et le composé fluoré peuvent être linéaires ou ramifiés, de préférence linéaires.

Selon un mode de réalisation, le composé chloré et le composé fluoré comportent un seul atome de carbone.

Selon un mode de réalisation, le composé chloré et le composé fluoré comportent deux atomes de carbone.

Selon un mode de réalisation, le composé chloré et le composé fluoré comportent trois atomes de carbone.

Selon un mode de réalisation, le composé chloré et le composé fluoré comportent quatre atomes de carbone.

Selon un mode de réalisation, le composé chloré et le composé fluoré comportent cinq atomes de carbone.

L'invention trouve notamment à s'appliquer pour les réactions de fluoration suivantes :
- fluoration du perchloroéthylène (PER) en pentafluoroéthane (HFC-125) ;
- fluoration du 1,1,1,2,3-pentachloropropane (HCC-240db) en 2-chloro-3,3,3-trifluoropropène (HCFO-1233xf) ;
- fluoration du 1,1,1,2,3-pentachloropropane (HCC-240db) en 2,3,3,3-tétrafluoropropène (HFO-1234yf) ;
- fluoration du 1,1,1,3,3-pentachloropropane (HCC-240fa) en 1,3,3,3-tétrafluoropropène (HFO-1234ze) ;
- fluoration du 1,1,1,3,3-pentachloropropane (HCC-240fa) en 1-chloro-3,3,3-trifluoropropène (HCFO-1233zd) ;
- fluoration du 2-chloro-3,3,3-trifluoropropène (HCFO-1233xf) en 2,3,3,3-tétrafluoropropène (HFO-1234yf);
- fluoration du 1,1,2,2,3-pentachloropropane (HCC-240aa) en 2,3,3,3-tétrafluoropropène (HFO-1234yf) ;
- fluoration du 1,1,2,2,3-pentachloropropane (HCC-240aa) en 2-chloro-3,3,3-trifluoropropène (HCFO-1233xf) ;
- fluoration du 2,3-dichloro-1,1,1-trifluoropropane (HCFC-243db) en 2,3,3,3-tétrafluoropropène (HFO-1234yf) ;
- fluoration du 2,3-dichloro-1,1,1-trifluoropropane (HCFC-243db) en 1-chloro-3,3,3-trifluoropropène (HCFO-1233zd) ;
- fluoration du 2,3-dichloro-1,1,1-trifluoropropane (HCFC-243db) en 1,3,3,3-tétrafluoropropène (HFO-1234ze) ;
- fluoration du 2,3-dichloro-1,1,1-trifluoropropane (HCFC-243db) en 2-chloro-3,3,3-trifluoropropène (HCFO-1233xf) ;
- fluoration du 1,1,2,3-tétrachloropropène (HCO-1230xa) en 2,3,3,3-tétrafluoropropène (HFO-1234yf) ;
- fluoration du 1,1,2,3-tétrachloropropène (HCO-1230xa) en 2-chloro-3,3,3-trifluoropropène (HCFO-1233xf) ;
- fluoration du 2,3,3,3-tetrachloropropène (HCO-1230xf) en 2,3,3,3-tétrafluoropropène (HFO-1234yf) ;
- fluoration du 2,3,3,3-tétrachloropropène (HCO-1230xf) en 2-chloro-3,3,3-trifluoropropène (HCFO-1233xf) ;
- fluoration du 1,1,3,3-tétrachloropropène (HCO-1230za) en 1-chloro-3,3,3-trifluoropropène (HCFO-1233zd) ;
- fluoration du 1,1,3,3-tétrachloropropène (HCO-1230za) en 1,3,3,3-tétrafluoropropène (HFO-1234ze) ;
- fluoration du 1,3,3,3-tétrachloropropène (HCO-1230zd) en 1-chloro-3,3,3-trifluoropropène (HCFO-1233zd) ;
- fluoration du 1,3,3,3-tétrachloropropène (HCO-1230zd) en 1,3,3,3-tétrafluoropropène (HFO-1234ze) ;
- fluoration du 1-chloro-3,3,3-trifluoropropène (HCFO-1233zd) en 1,3,3,3-tétrafluoropropène (HFO-1234ze) ;
- fluoration du 1,1,2-trichloroéthane en 1-chloro,2,2-difluoroéthane (HCFC-142) ;
- fluoration du 1,2-dichloroéthylène en 1-chloro-2,2-difluoroéthane (HCFC-142)

La conversion du composé chloré en composé fluoré peut être une conversion directe (avec une seule étape de réaction ou avec un seul ensemble de conditions réactionnelles) ou une conversion indirecte (avec deux ou plus de deux étapes de réaction ou en utilisant deux ou plus de deux ensembles de conditions réactionnelles).

La réaction de fluoration peut être effectuée :
- avec un rapport molaire HF / composé chloré de 3:1 à 150:1, de préférence de 4:1 à 100:1 et de manière plus particulièrement préférée de 5:1 à 50:1 ;
- avec un temps de contact de 1 à 100 s, de préférence 1 à 50 s et plus particulièrement 2 à 40 s (volume de catalyseur divisé par le flux entrant total, ajusté à la température et à la pression de fonctionnement) ;
- à une pression absolue allant de 0,1 à 50 bar, de préférence de 0,3 à 15 bar ;
- à une température (température du lit de catalyseur) de 100 à 500°C, de préférence de de 200 à 450°C, et plus particulièrement de 300 à 400°C.

Afin d'éviter une désactivation rapide du catalyseur lors de la réaction, un agent d'oxydation (par exemple de l'oxygène ou du chlore) peut être ajouté, par exemple dans un rapport molaire agent d'oxydation / composés organiques de 0,005 à 2, de préférence de 0,01 à 1,5. On peut par exemple utiliser un flux d'oxygène pur ou de chlore pur, ou un mélange oxygène / azote ou chlore / azote.

De manière préférée, on utilise un flux contenant de l'oxygène pour la mise en oeuvre de la fluoration (cette utilisation entraînant corrélativement l'apparition dans le flux de produits de contaminants tels que le fluorure de trifluoracétyle et les dérivés fluorés du phosgène).

Le catalyseur utilisé peut être par exemple à base d'un métal comprenant un oxyde de métal de transition ou un dérivé ou un halogénure ou un oxyhalogénure d'un tel métal. On peut citer par exemple FeCl₃, l'oxyfluorure de chrome, les oxydes de chrome (éventuellement soumis à des traitements de fluoration), les fluorures de chrome et leurs mélanges. D'autres catalyseurs possibles sont les catalyseurs supportés sur du carbone, les catalyseurs à base d'antimoine, les catalyseurs à base d'aluminium (par exemple AlF₃ et Al₂O₃, l'oxyfluorure d'alumine et le fluorure d'alumine).

On peut utiliser en général un oxyfluorure de chrome, un fluorure ou un oxyfluorure d'aluminium, ou un catalyseur supporté ou non contenant un métal tel que Cr, Ni, Fe, Zn, Ti, V, Zr, Mo, Ge, Sn, Pb, Mg, Sb.

On peut faire référence à cet égard au document WO 2007/079431 (en p.7, 1.1-5 et 28-32), au document EP 939071 (paragraphe [0022]), au document WO 2008/054781 (en p.9 l.22-p.10 l.34), et au document WO 2008/040969 (revendication 1), auxquels il est fait expressément référence.

Avant son utilisation, le catalyseur est de préférence soumis à une activation avec de l'air, de l'oxygène ou du chlore et/ou avec de l'HF.

Avant son utilisation, le catalyseur est de préférence soumis à une activation avec de l'air ou de l'oxygène et du HF à une température de 100 à 500°C, de préférence de 250 à 500°C et plus particulièrement de 300 à 400°C. La durée d'activation est de préférence de 1 à 200 h et plus particulièrement de 1 à 50 h.

Cette activation peut être suivie d'une étape d'activation de fluoration finale en présence d'un agent d'oxydation, d'HF et de composés organiques.

Le rapport molaire HF / composés organiques est de préférence de 2 à 40 et le rapport molaire agent d'oxydation / composés organiques est de préférence de 0,04 à 25. La température de l'activation finale est de préférence de 300 to 400°C and sa durée de préférence de 6 à 100 h.

Le catalyseur est de préférence à base de chrome et il s'agit plus particulièrement d'un catalyseur mixte comprenant du chrome.

Selon un mode de réalisation, on utilise un catalyseur mixte comprenant du chrome et du nickel. Le rapport molaire Cr / Ni (sur la base de l'élément métallique) est généralement de 0,5 à 5, par exemple de 0,7 à 2, par exemple d'environ 1. Le catalyseur peut contenir de 0,5 à 20 % en poids de chrome, et de 0,5 à 20 % en poids de nickel, de préférence de 2 à 10 % de chaque.

Le métal peut être présent sous forme métallique ou sous forme de dérivé, par exemple un oxyde, halogénure ou oxyhalogénure. Ces dérivés sont de préférence obtenus par activation du métal catalytique.

Le support est de préférence constitué avec de l'aluminium, par exemple de l'alumine, de l'alumine activée ou des dérivés d'aluminium, tels que les halogénures d'aluminium et les oxyhalogénures d'aluminium, par exemple décrits dans le document US 4,902,838, ou obtenus par le procédé d'activation décrit ci-dessus.

Le catalyseur peut comprendre du chrome et du nickel sous une forme activée ou non, sur un support qui a été soumis à une activation ou non.

On peut se reporter au document WO 2009/118628 (notamment en p.4, l.30-p.7 1.16), auquel il est fait expressément référence ici.

Un autre mode de réalisation préféré repose sur un catalyseur mixte contenant du chrome et au moins un élément choisi parmi Mg et Zn. Le rapport atomique de Mg ou Zn/Cr est de préférence de 0,01 à 5.

En faisant à présent référence à la figure 1, l'installation selon l'invention peut comprendre trois unités de traitement successives, à savoir une unité d'hydrolyse catalytique 2, une unité de lavage 4 et une unité d'adsorption d'impuretés 7, en amont d'une unité d'absorption adiabatique ou isotherme 9. En aval de l'unité d'absorption 9, une unité d'adsorption complémentaire 13 est prévue à titre optionnel.

Le flux gazeux à traiter, qui de préférence constitue une partie d'un flux de produits de réaction de fluoration catalytique (séparé par distillation), est amené par une conduite d'amenée de flux gazeux 1 à l'unité d'hydrolyse catalytique 2.

Dans cette unité d'hydrolyse catalytique 2, les composés fluoro-oxygénés du flux gazeux sont hydrolysés. Les principales réactions d'hydrolyse sont les suivantes :

COF₂ + H₂O → CO₂ + 2 HF

COFCl + H₂O → CO₂ + HCl + HF

CF₃COF + H₂O → CF₃COOH + HF

L'unité d'hydrolyse catalytique 2 est pourvue d'un lit catalytique, qui de préférence est un lit de charbon actif. La température de l'étape d'hydrolyse catalytique est de préférence de 100 à 200°C, notamment de 120 à 170°C, et plus particulièrement de 130 à 150°C. La pression est de préférence de 0,5 à 3 barg, notamment de 1 à 2 barg.

Le temps de séjour des espèces d'intérêt dans l'unité est de préférence de 1 s à 1 min, notamment de 2 s à 30 s, plus particulièrement de 4 s à 15 s, et tout particulièrement de 5 s à 10 s.

La quantité d'eau dans le mélange soumis à l'hydrolyse catalytique est ajustée de sorte que le rapport molaire de l'eau par rapport à la somme des composés fluoro-oxygénés soit supérieur à 1, de préférence supérieur ou égal à 2, ou à 3, ou à 4, ou à 5, ou à 6 ou à 6,5. Un apport d'eau peut être prévu si nécessaire.

Le taux d'hydrolyse (proportion molaire de composés fluoro-oxygénés hydrolysés) est de préférence supérieur à 90 %, plus particulièrement supérieur à 95 %, à 98 %, à 99 %, à 99,5 %, à 99,9 %, à 99,95 % ou à 99,99 %.

En sortie de l'unité d'hydrolyse catalytique 2, le flux gazeux est prélevé dans une conduite de collecte de flux gazeux hydrolysé 3. Celle-ci alimente une unité de lavage 4. L'unité de lavage 4 peut être une colonne à plateaux, telle qu'une colonne à plateaux perforés, ou à cloches, ou à clapets ou de type Dualflow®. Il peut également s'agir d'une colonne à garnissage. Le lavage du flux gazeux est effectué de préférence à contre-courant : le flux gazeux est alimenté en pied, et une solution acide est alimentée en tête, via une conduite d'amenée de solution acide 6.

A titre de solution acide, on peut notamment utiliser une solution d'HCl, à une concentration massique pouvant aller par exemple de 5 à 60 %, notamment de 10 à 50 %, plus préférentiellement de 20 à 45 % et en particulier de 30 à 35 %.

Le lavage par la solution acide est de préférence mis en oeuvre à une température de 5 à 50°C, et plus particulièrement de 7 à 40°C ; et/ou à une pression de 0,1 à 4 barg, de préférence de 0,3 à 2 barg, plus préférentiellement de 0,5 à 1,5 barg.

L'efficacité d'absorption du HF et du TFA dans la solution acide dépend du nombre de plateaux théoriques installés, du taux de reflux et de la température de la solution d'HCl concentrée. De manière générale, l'HF est plus facile à absorber que le TFA.

Un ajout d'acide borique au stade du lavage par la solution acide permet de complexer les ions fluorures. Par exemple, l'ajout de 2000 à 8000 ppm de H₃BO₃ permet d'améliorer l'efficacité d'absorption et d'atteindre des teneurs en TFA dans la solution commerciale d'HCl inférieure à 25 ppm. En d'autres termes, cette solution d'ajout d'acide borique permet d'installer une colonne présentant un nombre d'étages théoriques moindre et fonctionnant avec un taux de reflux inférieur, pour obtenir une même efficacité d'absorption d'HF et de TFA dans la solution HCl concentrée.

Au stade du lavage par la solution acide, la grande majorité de l'HF et du TFA du flux gazeux passe en solution et est donc éliminée via une conduite de collecte de solution acide usée 15 en pied de colonne. Une hydrolyse complémentaire des composés fluoro-oxygénés éventuellement restants dans le flux gazeux peut avoir lieu au stade du lavage.

Le flux gazeux lavé issu de l'unité de lavage 4 est récupéré via une conduite de collecte de flux gazeux lavé 5. Celle-ci alimente une unité d'adsorption d'impuretés 7, qui comprend un lit de charbon actif. Les impuretés adsorbées par le lit de charbon actif sont en premier lieu des composés organiques volatils (COV).

En effet, l'HCl gazeux peut être pollué par des composés organiques légers entraînés avec lui en tête de la distillation à de très faibles teneurs (ou à de plus fortes teneurs lors d'un dérèglement de la distillation par exemple). Ces composés organiques légers peuvent comprendre le trifluorométhane (F23), le pentafluoroéthane (F125), le chloropentafluoroéthane (F115), le 1,1,1-trifluoroéthane (F143a), le fluorométhane (F41), la trifluoropropyne, etc. Par exemple, le F125 et le F115 forment un azéotrope avec l'HCI.

L'étape d'adsorption sur lit de charbon actif peut être mise en oeuvre dans des gammes de pression et de température qui ont déjà été indiquées ci-dessus en relation avec l'étape de lavage par une solution acide.

En sortie de l'unité d'adsorption d'impuretés 7, le flux gazeux purifié est récupéré dans une conduite de collecte de flux purifié 8, qui est connectée en entrée d'une unité d'absorption adiabatique ou isotherme 9. L'unité d'absorption 9 permet d'absorber le HCl du flux gazeux dans une solution aqueuse, apportée par une conduite d'amenée de solution aqueuse 10. Cette solution aqueuse peut être simplement de l'eau déminéralisée, ou alternativement peut être une solution acide.

Généralement, l'unité d'absorption 9 comporte une colonne de mise en contact à contre-courant, la solution aqueuse étant fournie en tête et le flux gazeux en pied.

La réaction d'absorption de l'HCl dans l'eau étant exothermique, il est préférable de limiter la pression à laquelle cette opération est conduite. En général, la pression est inférieure à 2 barg et de préférence inférieure à 1,5 barg. De cette façon la température d'absorption n'excède pas 130°C, et de préférence 120°C. Pour résister à la corrosion, la colonne peut être en graphite ou bien en acier revêtu de polytétrafluoroéthylène (PTFE). Les internes de colonne peuvent être par exemple soit en graphite soit en polyfluorure de vinylidène (PVDF).

Un flux gazeux désacidifié est récolté en tête, via une conduite de collecte de flux gazeux désacidifié 11. Ce flux peut être soit rejeté à l'atmosphère via une colonne de sécurité de neutralisation, soit envoyé vers un incinérateur.

Une solution d'HCl est récoltée en pied, via une conduite de collecte de solution d'acide chlorhydrique 12. La concentration massique d'HCl dans la solution peut être de 5 à 50 %, de préférence de 15 à 40 %, et plus particulièrement de 30 à 35 %. Une partie de cette solution peut être utilisée comme solution de lavage dans l'unité de lavage 4. Dans ce cas, la conduite d'amenée de solution acide 6 peut être branchée sur la conduite de collecte de solution d'acide chlorhydrique 12. La proportion de solution d'HCl ainsi utilisée pour les besoins du lavage peut représenter de 2 à 15 % en masse, de préférence de 5 à 10 %.

Si la pureté de la solution d'HCl collectée n'est pas suffisante, et notamment si la teneur en HF reste supérieure au seuil souhaité, il est possible de procéder à une autre étape de traitement, à savoir une étape d'adsorption dans une unité d'adsorption complémentaire 13, alimentée par la conduite de collecte de solution d'acide chlorhydrique 12. Cette unité d'adsorption complémentaire 13 peut comprendre par exemple un gel de silice.

La température de la solution d'HCl doit être aussi faible que possible, et par exemple inférieure ou égale à 35°C, car l'adsorption sur le gel de silice est exothermique. Au-dessus de cette température, l'efficacité d'adsorption diminue fortement. Le temps de contact est compris entre quelques minutes et quelques heures (de préférence entre 10 et 60 min). Les vitesses de passage sont lentes et comprises entre 1 et 20 m/h et de préférence entre 3 et 10 m/h. La pression de fonctionnement est de quelques bars (de 1 à 7 barg et de préférence de 1 à 5 barg). Le gel de silice comporte typiquement une taille de pores de 50 Å, alors que les gels classiques ont en général des tailles de pores de 20 Å au maximum. La teneur en fluorures de la solution d'HCl en entrée est de préférence inférieure ou égale à 100 ppm pour éviter tout risque de dégradation du gel de silice. Il s'agit donc d'une opération de finition. Après cette étape d'adsorption complémentaire sur gel de silice, il est possible d'atteindre des teneurs en HF inférieures à 1 ppm dans la solution d'HCl.

En sortie de l'unité d'adsorption complémentaire 13, on récupère une solution d'HCl purifiée, dans une conduite de collecte de solution d'acide chlorhydrique purifiée 14.

La solution d'HCl (ou solution d'HCl purifiée) récupérée à l'issue du procédé de l'invention peut être valorisée commercialement.

De préférence, la teneur maximale en fluorures dans cette solution d'HCl (ou solution d'HCl purifiée) récupérée à l'issue du procédé, est de 5 ppm, voire de 1 ppm. La teneur maximale en composés organiques fluoro-oxygénés (et notamment en TFA) peut être par exemple de 25 ppm.

### EXEMPLES

Le montage comprend un four d'hydrolyse alimenté à l'aide d'une pompe et équipé d'un compteur volumétrique de gaz spécifique pour les gaz corrosifs permettant la mesure du gaz entrant. Le four est également alimenté en eau par une pompe adaptée à notre gamme de débit. L'enceinte interne du four est en graphite (température maximum de 200°C) et contient un lit de charbon actif. Le four utilise un système de chauffe par induction électromagnétique généré par un inducteur en cuivre. Tous les matériaux utilisés pour la construction du four sont compatibles avec les gaz corrosifs manipulés.

Le flux gazeux résultant est envoyé au pied d'une colonne à plateaux. Le flux d'acide chlorhydrique à purifier peut également être envoyé directement à la colonne à plateaux sans passer par le four d'hydrolyse. Ce dispositif permet de mettre en évidence les avantages du four d'hydrolyse. La colonne a une hauteur de 1,20 mètres et cinq centimètres de diamètre. Elle comporte vingt plateaux perforés (200 perforations par plateau, chacune faisant 1,75 mm de diamètre). Avec une hauteur moyenne de 1 cm de liquide par plateau, on arrive à un volume de rétention théorique de 390 ml de liquide. L'écartement entre les plateaux est égal au diamètre de la colonne. Chaque plateau comporte un déversoir permettant la circulation du liquide vers le pied de colonne tandis que les perforations autorisent le passage du gaz vers la tête de colonne. Le matériau préféré pour la colonne est le PVDF. La colonne est alimentée en acide chlorhydrique liquide commerciale 37% en poids qui va permettre de laver et purifier le gaz anhydre après son passage ou non dans le four d'hydrolyse.

Le dispositif est installé en dérivation sur un atelier industriel de manière à traiter un gaz réel. Le débit de gaz à purifier sur cette dérivation est d'environ 300l/h. Ce gaz est issu d'une première colonne à distiller qui suit l'étape réactionnelle catalytique de fluoration effectuée en phase gazeuse en présence d'oxygène. Dans cette colonne à distiller, le produit organique majoritaire est obtenu en pied de colonne tandis que l'HCl anhydre (76% vol.) et l'air (23% vol.) sont séparés en tête de colonne. Ce flux d'HCl contient également des impuretés organiques fluorées (1%), les plus génantes sont CF₃COF et COF₂. Ces impuretés se retrouvent ensuite dans les solutions d'acide chlorhydrique (sous forme de fluorure et/ou d'acide trifluoroacétique, appelé aussi TFA) après leur absorption et limitent la valorisation des solutions d'acide chlorhydrique obtenues. Les réactions d'hydrolyse considérées sont :

CF₃COF + H₂O → CF₃COOH (Acide trifluoroacétique) + HF COF₂ + H₂O → CO₂ + 2 HF

L'analyse des impuretés contenues dans le gaz d'acide chlorhydrique avant et après traitement est effectuée par chromatographie ionique d'une solution aqueuse dans laquelle le gaz a barboté pendant un temps et avec un débit défini. La chromatographie ionique permet d'évaluer avec précision les quantités d'ions fluorures et les ions trifluoroacétates, CF₃COO⁻. Les taux d'impuretés sont exprimés en ppm dans une solution d'acide chlorhydrique à 33% pour faciliter les comparaisons.

### Exemple 1 : sans utilisation du four d'hydrolyse

Un débit de 265l/h de gaz à purifier contenant majoritairement de l'acide chlorhydrique anhydre est alimenté au pied de la colonne de lavage à plateaux perforés. 332g/h de solution d'acide chlorhydrique liquide à 37% sont alimentés en tête de colonne pour le lavage. Après remplissage et équilibrage des plateaux, l'essai est maintenu pendant 5 heures. Les résultats obtenus sur la teneur en impuretés dans le gaz anhydre sont donnés dans le tableau 1.

### Exemple 2 : avec utilisation du four d'hydrolyse

Un débit de 295l/h de gaz à purifier contenant majoritairement de l'acide chlorhydrique anhydre est alimenté au four d'hydrolyse. La température du four est maintenue vers 100°C. Le four est alimenté en eau avec un débit de 83,5g/h. Le flux gazeux résultant est ensuite alimenté au pied de la colonne de lavage à plateaux perforés. 244g/h de solution d'acide chlorhydrique liquide à 37% sont alimentés en tête de colonne pour le lavage. Après remplissage et équilibrage des plateaux, l'essai est maintenu pendant 4h30 heures. Les résultats obtenus sur la teneur en impuretés dans le gaz anhydre sont donnés dans le tableau 1.

**Tableau 1- effet du traitement de purification sans et avec four d'hydrolyse.**

| | Exemple 1 | | Exemple 2 | |
|---|---|---|---|---|
| ppm (poids) | Entrée gaz | Sortie gaz | Entrée gaz | Sortie gaz |
| TFA/HCl 33% | 2766 | 1624 | 2279 | 1 |
| F/HCl 33% | 894 | 431 | 779 | 4 |

L'étape limitante semble bien être l'hydrolyse des molécules qui se fait difficilement dans la colonne de lavage. Par contre, une fois cette hydrolyse achevée, l'élimination des impuretés par le procédé dit « strong-acid-wash » ne semble plus poser de problèmes. La combinaison des deux étapes est donc nécessaire pour l'obtention d'une bonne pureté de l'HCl.

## Revendications

1. Procédé de traitement d'un flux gazeux contenant de l'acide chlorhydrique, de l'acide fluorhydrique et des composés fluoro-oxygénés, dans lequel le flux gazeux est successivement soumis à :
- une étape d'hydrolyse catalytique des composés fluoro-oxygénés;
- une étape de lavage par une solution acide ;
- une étape d'adsorption d'impuretés par du charbon actif ;
- une étape d'absorption adiabatique ou isotherme de l'acide chlorhydrique dans une solution aqueuse, permettant de collecter une solution d'acide chlorhydrique.

2. Procédé selon la revendication 1, dans lequel l'étape d'hydrolyse catalytique est effectuée sur un lit de charbon actif.

3. Procédé selon la revendication 1 ou 2, dans lequel la solution acide utilisée lors de l'étape de lavage est une solution d'acide chlorhydrique et de préférence provient de la solution d'acide chlorhydrique collectée à l'issue de l'étape d'absorption adiabatique ou isotherme.

4. Procédé selon l'une des revendications 1 à 3, comprenant en outre :
- une étape de mise en contact de la solution d'acide chlorhydrique avec un gel de silice.

5. Procédé selon l'une des revendications 1 à 4, dans lequel les composés fluoro-oxygénés comprennent du difluorure de carbonyle, du chlorofluorocarbonyle, du fluorure de trifluoroacétyle et/ou de l'acide trifluoroacétique ; et dans lequel, de préférence, le flux gazeux comprend au moins 50 mg/L, notamment au moins 100 mg/L, voire au moins 200 mg/L de fluorure de trifluoroacétyle et/ou acide trifluoroacétique.

6. Procédé selon l'une des revendications 1 à 5, dans lequel de l'acide borique est ajouté à la solution acide utilisée pour l'étape de lavage.

7. Procédé selon l'une des revendications 1 à 6, dans lequel le flux gazeux est un flux issu d'une réaction de fluoration catalytique d'au moins un composé chloré en au moins un composé fluoré, ledit flux étant de préférence collecté en sortie d'une distillation d'un flux de produits de la réaction de fluoration catalytique.

8. Procédé selon la revendication 7, dans lequel la réaction de fluoration catalytique est effectuée en présence d'oxygène.

9. Procédé selon la revendication 7 ou 8, dans lequel :
- le composé chloré est un chlorocarbure, un hydrochlorocarbure, un chlorofluorocarbure, un hydrochlorofluorocarbure, une chlorooléfine, une hydrochlorooléfine, une chlorofluorooléfine ou une hydrochlorofluorooléfine ; et dans lequel le composé fluoré est un fluorocarbure, un hydrofluorocarbure, un chlorofluorocarbure, un hydrochlorofluorocarbure, une fluorooléfine, une hydrofluorooléfine, une chlorofluorooléfine ou une hydrochlorofluorooléfine ;
- de préférence le composé chloré est choisi parmi le 1,1,2-trichloroéthane, le 1,1,1,2,3-pentachloropropane, le 1,1,1,3,3-pentachloropropane, le 1,1,2,2,3-pentachloropropane, le 2,3-dichloro-1,1,1-trifluoropropane, le perchloroéthylène, le 1,2-dichloroéthylène, le 1,1,2,3-tétrachloropropène, le 2,3,3,3-tetrachloropropène, le 1,1,3,3-tétrachloropropène, le 1,3,3,3-tétrachloropropène, le 2-chloro-3,3,3-trifluoropropène, le 1-chloro-3,3,3-trifluoropropène et leurs mélanges ; et
- de préférence le composé fluoré est choisi parmi le pentafluoroéthane, le 1-chloro,2,2-difluoroéthane, le 1,3,3,3-tétrafluoropropène, le 2,3,3,3-tétrafluoropropène, le 2-chloro-3,3,3-trifluoropropène, le 1-chloro-3,3,3-trifluoropropène et leurs mélanges.

10. Procédé de préparation d'un composé fluoré comprenant :
- la fourniture d'un composé chloré et d'acide fluorhydrique ;
- la réaction catalytique du composé chloré avec l'acide fluorhydrique et la collecte d'un flux de produits bruts ;
- la séparation du flux de produits bruts, de préférence par distillation, permettant de récupérer d'une part un flux de composé fluoré, et d'autre part un flux gazeux contenant principalement de l'acide chlorhydrique, ainsi que des quantités minoritaires d'acide fluorhydrique et de composés fluoro-oxygénés ;
- le traitement du flux gazeux contenant de l'acide chlorhydrique, de l'acide fluorhydrique et des composés fluoro-oxygénés selon l'une des revendications 1 à 9.

11. Procédé selon la revendication 10, dans lequel la réaction catalytique est mise en oeuvre en présence d'oxygène.

12. Procédé selon la revendication 10 ou 11, dans lequel :
- le composé chloré est un chlorocarbure, un hydrochlorocarbure, un chlorofluorocarbure, un hydrochlorofluorocarbure, une chlorooléfine, une hydrochlorooléfine, une chlorofluorooléfine ou une hydrochlorofluorooléfine ; et dans lequel le composé fluoré est un fluorocarbure, un hydrofluorocarbure, un chlorofluorocarbure, un hydrochlorofluorocarbure, une fluorooléfine, une hydrofluorooléfine, une chlorofluorooléfine ou une hydrochlorofluorooléfine ;
- de préférence le composé chloré est choisi parmi le 1,1,2-trichloroéthane, le 1,1,1,2,3-pentachloropropane, le 1,1,1,3,3-pentachloropropane, le 1,1,2,2,3-pentachloropropane, le 2,3-dichloro-1,1,1-trifluoropropane, le perchloroéthylène, le 1,2-dichloroéthylène, le 1,1,2,3-tétrachloropropène, le 2,3,3,3-tetrachloropropène, le 1,1,3,3-tétrachloropropène, le 1,3,3,3-tétrachloropropène, le 2-chloro-3,3,3-trifluoropropène, le 1-chloro-3,3,3-trifluoropropène et leurs mélanges ; et
- de préférence le composé fluoré est choisi parmi le pentafluoroéthane, le 1-chloro,2,2-difluoroéthane, le 1,3,3,3-tétrafluoropropène, le 2,3,3,3-tétrafluoropropène, le 2-chloro-3,3,3-trifluoropropène, le 1-chloro-3,3,3-trifluoropropène et leurs mélanges.

13. Installation de traitement d'un flux gazeux contenant de l'acide chlorhydrique, de l'acide fluorhydrique et des composés fluoro-oxygénés, comprenant :
- une conduite d'amenée de flux gazeux contenant de l'acide chlorhydrique, de l'acide fluorhydrique et des composés fluoro-oxygénés (1), alimentant une unité d'hydrolyse catalytique (2) ;
- une unité de lavage (4), alimentée d'une part par une conduite de collecte de flux gazeux hydrolysé (3) issue de l'unité d'hydrolyse catalytique (2), et d'autre part par une conduite d'amenée de solution acide (6) ;
- une unité d'adsorption d'impuretés (7), comprenant un lit de charbon actif, alimentée par une conduite de collecte de flux gazeux lavé (5) issue de l'unité de lavage ;
- une unité d'absorption adiabatique ou isotherme (9), alimentée d'une part par une conduite de collecte de flux purifié (8) issue de l'unité d'adsorption et d'autre part par une conduite d'amenée de solution aqueuse (10) ;
- une conduite de collecte de solution d'acide chlorhydrique (12) en sortie de l'unité d'absorption adiabatique ou isotherme (9).

14. Installation selon la revendication 13, dans laquelle l'unité d'hydrolyse catalytique (2) comprend un lit de charbon actif.

15. Installation selon la revendication 13 ou 14, dans laquelle la conduite d'amenée de solution acide (6) est issue directement ou non de la conduite de collecte de solution d'acide chlorhydrique (12).

16. Installation selon l'une des revendications 13 à 15, comprenant en outre :
- une unité d'adsorption complémentaire (13) comprenant un gel de silice, alimentée par la conduite de collecte de solution d'acide chlorhydrique (12) ; et
- une conduite de collecte de solution d'acide chlorhydrique purifiée (14) issue de l'unité d'adsorption supplémentaire (13).

17. Installation selon l'une des revendications 13 à 16, comprenant un apport de solution d'acide borique à l'unité de lavage (4).

18. Installation selon l'une des revendications 13 à 17, dans laquelle la conduite d'amenée de flux gazeux contenant de l'acide chlorhydrique, de l'acide fluorhydrique et des composés fluoro-oxygénés (1) est issue d'une unité de distillation; l'unité de distillation étant de préférence alimentée par une conduite de collecte de produits bruts en sortie d'un réacteur catalytique.

19. Installation selon la revendication 18, dans laquelle le réacteur catalytique est alimenté par une conduite d'amenée de composé chloré et une conduite d'amenée d'acide fluorhydrique, et la conduite de collecte de produits bruts transporte un flux comprenant un composé fluoré, et dans laquelle de préférence :
- le composé chloré est un chlorocarbure, un hydrochlorocarbure, un chlorofluorocarbure, un hydrochlorofluorocarbure, une chlorooléfine, une hydrochlorooléfine, une chlorofluorooléfine ou une hydrochlorofluorooléfine ; et dans lequel le composé fluoré est un fluorocarbure, un hydrofluorocarbure, un chlorofluorocarbure, un hydrochlorofluorocarbure, une fluorooléfine, une hydrofluorooléfine, une chlorofluorooléfine ou une hydrochlorofluorooléfine ;
- plus particulièrement le composé chloré est choisi parmi le 1,1,2-trichloroéthane, le 1,1,1,2,3-pentachloropropane, le 1,1,1,3,3-pentachloropropane, le 1,1,2,2,3-pentachloropropane, le 2,3-dichloro-1,1,1-trifluoropropane, le perchloroéthylène, le 1,2-dichloroéthylène, le 1,1,2,3-tétrachloropropène, le 2,3,3,3-tetrachloropropène, le 1,1,3,3-tétrachloropropène, le 1,3,3,3-tétrachloropropène, le 2-chloro-3,3,3-trifluoropropène, le 1-chloro-3,3,3-trifluoropropène et leurs mélanges ; et
- plus particulièrement le composé fluoré est choisi parmi le pentafluoroéthane, le 1-chloro,2,2-difluoroéthane, le 1,3,3,3-tétrafluoropropène, le 2,3,3,3-tétrafluoropropène, le 2-chloro-3,3,3-trifluoropropène, le 1-chloro-3,3,3-trifluoropropène et leurs mélanges.

20. Installation selon la revendication 18 ou 19, comprenant un apport d'oxygène vers le réacteur catalytique.

## Patentansprüche

1. Verfahren zur Behandlung eines Gasstroms, der Salzsäure, Fluorwasserstoffsäure und fluor-/sauerstoffhaltige Verbindungen umfasst, wobei der Gasstrom nacheinander Folgendem unterzogen wird:
- einem Schritt der katalytischen Hydrolyse der fluor-/sauerstoffhaltigen Verbindungen;
- einem Waschschritt mit einer sauren Lösung;
- einem Schritt der Adsorption von Verunreinigungen durch Aktivkohle;
- einem Schritt der adiabatischen oder isothermen Absorption der Salzsäure in einer wässrigen Lösung, wodurch eine Salzsäurelösung gesammelt werden kann.

2. Verfahren nach Anspruch 1, wobei der Schritt der katalytischen Hydrolyse auf einem Aktivkohlebett durchgeführt wird.

3. Verfahren nach Anspruch 1 oder 2, wobei die während des Waschschritts verwendete saure Lösung eine Salzsäurelösung ist und vorzugsweise von der Salzsäurelösung herrührt, die am Ende des Schritts der adiabatischen oder isothermen Absorption gesammelt wird.

4. Verfahren nach einem der Ansprüche 1 bis 3, das außerdem Folgendes umfasst:
- einen Schritt des Inkontaktbringens der Salzsäurelösung mit einem Siliziumdioxidgel.

5. Verfahren nach einem der Ansprüche 1 bis 4, wobei die fluor-/sauerstoffhaltigen Verbindungen Carbonyldifluorid, Carbonylchloridfluorid, Trifluoracetylfluorid und/oder Trifluoressigsäure umfassen und wobei der Gasstrom vorzugsweise mindestens 50 mg/l, insbesondere mindestens 100 mg/l, sogar mindestens 200 mg/l Trifluoracetylfluorid und/oder Trifluoressigsäure umfasst.

6. Verfahren nach einem der Ansprüche 1 bis 5, wobei Borsäure zu der für den Waschschritt verwendeten sauren Lösung zugegeben wird.

7. Verfahren nach einem der Ansprüche 1 bis 6, wobei der Gasstrom ein Strom ist, der aus einer katalytischen Fluorierungsreaktion von mindestens einer chlorhaltigen Verbindung in mindestens eine fluorhaltige Verbindung herrührt, wobei der Strom vorzugsweise am Ausgang einer Destillation eines Produktstroms der katalytischen Fluorierungsreaktion gesammelt wird.

8. Verfahren nach Anspruch 7, wobei die katalytische Fluorierungsreaktion in Gegenwart von Sauerstoff durchgeführt wird.

9. Verfahren nach Anspruch 7 oder 8, wobei:
- die chlorhaltige Verbindung ein Chlorkohlenstoff, ein Chlorkohlenwasserstoff, ein Chlorfluorkohlenstoff, ein Chlorfluorkohlenwasserstoff, ein Chlorolefin, ein Hydrochlorolefin, ein Chlorfluorolefin oder ein Hydrochlorfluorolefin ist und wobei die fluorhaltige Verbindung ein Fluorkohlenstoff, ein Fluorkohlenwasserstoff, ein Chlorfluorkohlenstoff, ein Chlorfluorkohlenwasserstoff, ein Fluorolefin, ein Hydrofluorolefin, ein Chlorfluorolefin oder ein Hydrochlorfluorolefin ist;
- wobei die chlorhaltige Verbindung vorzugsweise aus 1,1,2-Trichlorethan, 1,1,1,2,3-Pentachlorpropan, 1,1,1,3,3-Pentachlorpropan, 1,1,2,2,3-Pentachlorpropan, 2,3-Dichlor-1,1,1-trifluorpropan, Perchlorethylen, 1,2-Dichlorethylen, 1,1,2,3-Tetrachlorpropen, 2,3,3,3-Tetrachlorpropen, 1,1,3,3-Tetrachlorpropen, 1,3,3,3-Tetrachlorpropen, 2-Chlor-3,3,3-trifluorpropen, 1-Chlor-3,3,3-trifluorpropen und deren Gemischen ausgewählt ist und
- die fluorhaltige Verbindung vorzugsweise aus Pentafluorethan, 1-Chlor-2,2-difluorethan, 1,3,3,3-Tetrafluorpropen, 2,3,3,3-Tetrafluorpropen, 2-Chlor-3,3,3-trifluorpropen, 1-Chlor-3,3,3-trifluorpropen und deren Gemischen ausgewählt ist.

10. Verfahren zur Herstellung einer fluorhaltigen Verbindung, das Folgendes umfasst:
- Beschickung mit einer chlorhaltigen Verbindung und Fluorwasserstoffsäure;
- katalytische Reaktion der chlorhaltigen Verbindung mit der Fluorwasserstoffsäure und Sammeln eines Rohproduktestroms;
- Trennen des Rohproduktestroms, vorzugsweise durch Destillation, wodurch einerseits ein Strom der fluorhaltigen Verbindung und andererseits ein Gasstrom gesammelt werden kann, der hauptsächlich Salzsäure sowie kleinere Mengen an Fluorwasserstoffsäure und fluor-/sauerstoffhaltigen Verbindungen enthält;
- Behandeln des Gasstroms, der die Salzsäure, die Fluorwasserstoffsäure und die fluorhaltigen/sauerstoffhaltigen Verbindungen enthält, nach einem der Ansprüche 1 bis 9.

11. Verfahren nach Anspruch 10, wobei die katalytische Reaktion in Gegenwart von Sauerstoff durchgeführt wird.

12. Verfahren nach Anspruch 10 oder 11, wobei:
- die chlorhaltige Verbindung ein Chlorkohlenstoff, ein Chlorkohlenwasserstoff, ein Chlorfluorkohlenstoff, ein Chlorfluorkohlenwasserstoff, ein Chlorolefin, ein Hydrochlorolefin, ein Chlorfluorolefin oder ein Hydrochlorfluorolefin ist und wobei die fluorhaltige Verbindung ein Fluorkohlenstoff, ein Fluorkohlenwasserstoff, ein Chlorfluorkohlenstoff, ein Chlorfluorkohlenwasserstoff, ein Fluorolefin, ein Hydrofluorolefin, ein Chlorfluorolefin oder ein Hydrochlorfluorolefin ist;
- wobei die chlorhaltige Verbindung vorzugsweise aus 1,1,2-Trichlorethan, 1,1,1,2,3-Pentachlorpropan, 1,1,1,3,3-Pentachlorpropan, 1,1,2,2,3-Pentachlorpropan, 2,3-Dichlor-1,1,1-trifluorpropan, Perchlorethylen, 1,2-Dichlorethylen, 1,1,2,3-Tetrachlorpropen, 2,3,3,3-Tetrachlorpropen, 1,1,3,3-Tetrachlorpropen, 1,3,3,3-Tetrachlorpropen, 2-Chlor-3,3,3-trifluorpropen, 1-Chlor-3,3,3-trifluorpropen und deren Gemischen ausgewählt ist und
- die fluorhaltige Verbindung vorzugsweise aus Pentafluorethan, 1-Chlor-2,2-difluorethan, 1,3,3,3-Tetrafluorpropen, 2,3,3,3-Tetrafluorpropen, 2-Chlor-3,3,3-trifluorpropen, 1-Chlor-3,3,3-trifluorpropen und deren Gemischen ausgewählt ist.

13. Anlage zur Behandlung eines Salzsäure, Fluorwasserstoffsäure und fluor-/sauerstoffhaltige Verbindungen umfassenden Gasstroms, die Folgendes umfasst:
- eine Leitung zum Zuführen eines Salzsäure, Fluorwasserstoffsäure und fluor-/sauerstoffhaltige Verbindungen enthaltenden Gasstroms (1), mit dem eine Einrichtung zur katalytischen Hydrolyse (2) beschickt wird;
- eine Wascheinrichtung (4), die einerseits durch eine Leitung zum Sammeln von hydrolysiertem Gasstrom (3), der aus der Einrichtung zur katalytischen Hydrolyse (2) stammt, und andererseits durch eine Leitung zum Zuführen von saurer Lösung (6) beschickt wird;
- eine Einrichtung zur Adsorption von Verunreinigungen (7), die ein Aktivkohlebett umfasst und durch eine Leitung zum Sammeln des gewaschenen Gasstroms (5), der aus der Wascheinrichtung stammt, beschickt wird;
- eine Einrichtung zur adiabatischen oder isothermen Absorption (9), die einerseits durch eine Leitung zum Sammeln des gereinigten Stroms (8), der aus der Adsorptionseinrichtung stammt, und andererseits durch eine Leitung zur Zuführung von wässriger Lösung (10) beschickt wird;
- eine Leitung zum Sammeln von Salzsäurelösung (12) am Ausgang der Einrichtung zur adiabatischen oder isothermen Absorption (9).

14. Anlage nach Anspruch 13, wobei die Einrichtung zur katalytischen Hydrolyse (2) ein Aktivkohlebett umfasst.

15. Anlage nach Anspruch 13 oder 14, wobei die Leitung zur Zuführung von saurer Lösung (6) direkt oder indirekt von der Leitung zum Sammeln von Salzsäurelösung (12) stammt.

16. Anlage nach einem der Ansprüche 13 bis 15, die außerdem Folgendes umfasst:
- eine zusätzliche Adsorptionseinrichtung (13), die ein Siliziumdioxidgel umfasst und durch die Leitung zum Sammeln von Salzsäurelösung (12) beschickt wird; und
- eine Leitung zum Sammeln von gereinigter Salzsäurelösung (14), die von der zusätzlichen Adsorptionseinrichtung (13) stammt.

17. Anlage nach einem der Ansprüche 13 bis 16, die eine Zufuhr von Borsäurelösung zur Wascheinrichtung (4) umfasst.

18. Anlage nach einem der Ansprüche 13 bis 17, wobei die Leitung zur Zuführung eines Salzsäure, Fluorwasserstoffsäure und fluor-/sauerstoffhaltige Verbindungen enthaltenden Gasstroms (1) aus einer Destillationseinrichtung stammt, wobei die Destillationseinrichtung vorzugsweise über eine Leitung zum Sammeln von Rohprodukten am Ausgang eines katalytischen Reaktors beschickt wird.

19. Anlage nach Anspruch 18, wobei der katalytische Reaktor durch eine Leitung zur Zuführung von chlorhaltiger Verbindung und eine Leitung zur Zuführung von Fluorwasserstoffsäure beschickt wird und die Leitung zum Sammeln von Rohprodukten einen Strom transportiert, der eine fluorhaltige Verbindung umfasst, und wobei vorzugsweise:
- die chlorhaltige Verbindung ein Chlorkohlenstoff, ein Chlorkohlenwasserstoff, ein Chlorfluorkohlenstoff, ein Chlorfluorkohlenwasserstoff, ein Chlorolefin, ein Hydrochlorolefin, ein Chlorfluorolefin oder ein Hydrochlorfluorolefin ist und wobei die fluorhaltige Verbindung ein Fluorkohlenstoff, ein Fluorkohlenwasserstoff, ein Chlorfluorkohlenstoff, ein Chlorfluorkohlenwasserstoff, ein Fluorolefin, ein Hydrofluorolefin, ein Chlorfluorolefin oder ein Hydrochlorfluorolefin ist;
- die chlorhaltige Verbindung insbesondere aus 1,1,2-Trichlorethan, 1,1,1,2,3-Pentachlorpropan, 1,1,1,3,3-Pentachlorpropan, 1,1,2,2,3-Pentachlorpropan, 2,3-Dichlor-1,1,1-trifluorpropan, Perchlorethylen, 1,2-Dichlorethylen, 1,1,2,3-Tetrachlorpropen, 2,3,3,3-Tetrachlorpropen, 1,1,3,3-Tetrachlorpropen, 1,3,3,3-Tetrachlorpropen, 2-Chlor-3,3,3-trifluorpropen, 1-Chlor-3,3,3-trifluorpropen und deren Gemischen ausgewählt ist und
- die fluorhaltige Verbindung insbesondere aus Pentafluorethan, 1-Chlor-2,2-difluorethan, 1,3,3,3-Tetrafluorpropen, 2,3,3,3-Tetrafluorpropen, 2-Chlor-3,3,3-trifluorpropen, 1-Chlor-3,3,3-trifluorpropen und deren Gemischen ausgewählt ist.

20. Anlage nach Anspruch 18 oder 19, die eine Zufuhr von Sauerstoff zum katalytischen Reaktor umfasst.

## Claims

1. Process for the treatment of a gas stream comprising hydrochloric acid, hydrofluoric acid and fluorinated/oxygenated compounds, in which the gas stream is successively subjected to:
- a stage of catalytic hydrolysis of the fluorinated/oxygenated compounds;
- a stage of washing with an acid solution;
- a stage of adsorption of impurities by active charcoal;
- a stage of adiabatic or isothermal absorption of the hydrochloric acid in an aqueous solution, making it possible to collect hydrochloric acid solution.

2. Process according to Claim 1, in which the catalytic hydrolysis stage is carried out on a bed of active charcoal.

3. Process according to Claim 1 or 2, in which the acid solution used during the washing stage is a hydrochloric acid solution and preferably originates from the hydrochloric acid solution collected on conclusion of the adiabatic or isothermal absorption stage.

4. Process according to one of Claims 1 to 3, additionally comprising:
- a stage of bringing the hydrochloric acid solution into contact with a silica gel.

5. Process according to one of Claims 1 to 4, in which the fluorinated/oxygenated compounds comprise carbonyl difluoride, carbonyl chloride fluoride, trifluoroacetyl fluoride and/or trifluoroacetic acid and in which, preferably, the gas stream comprises at least 50 mg/l, in particular at least 100 mg/l, indeed even at least 200 mg/l, of trifluoroacetyl fluoride and/or trifluoroacetic acid.

6. Process according to one of Claims 1 to 5, in which boric acid is added to the acid solution used for the washing stage.

7. Process according to one of Claims 1 to 6, in which the gas stream is a stream resulting from a catalytic fluorination reaction of at least one chlorinated compound to give at least one fluorinated compound, said stream preferably being collected at the outlet of a distillation of a stream of products from the catalytic fluorination reaction.

8. Process according to Claim 7, in which the catalytic fluorination reaction is carried out in the presence of oxygen.

9. Process according to Claim 7 or 8, in which:
- the chlorinated compound is a chlorocarbon, a hydrochlorocarbon, a chlorofluorocarbon, a hydrochlorofluorocarbon, a chloroolefin, a hydrochloroolefin, a chlorofluoroolefin or a hydrochlorofluoroolefin and in which the fluorinated compound is a fluorocarbon, a hydrofluorocarbon, a chlorofluorocarbon, a hydrochlorofluorocarbon, a fluoroolefin, a hydrofluoroolefin, a chlorofluoroolefin or a hydrochlorofluoroolefin;
- preferably, the chlorinated compound is chosen from 1,1,2-trichloroethane, 1,1,1,2,3-pentachloropropane, 1,1,1,3,3-pentachloropropane, 1,1,2,2,3-pentachloropropane, 2,3-dichloro-1,1,1-trifluoropropane, perchloroethylene, 1,2-dichloroethylene, 1,1,2,3-tetrachloropropene, 2,3,3,3-tetrachloropropene, 1,1,3,3-tetrachloropropene, 1,3,3,3-tetrachloropropene, 2-chloro-3,3,3-trifluoropropene, 1-chloro-3,3,3-trifluoropropene and their mixtures; and
- preferably, the fluorinated compound is chosen from pentafluoroethane, 1-chloro-2,2-difluoroethane, 1,3,3,3-tetrafluoropropene, 2,3,3,3-tetrafluoropropene, 2-chloro-3,3,3-trifluoropropene, 1-chloro-3,3,3-trifluoropropene and their mixtures.

10. Process for the preparation of a fluorinated compound, comprising:
- the provision of a chlorinated compound and of hydrofluoric acid;
- the catalytic reaction of the chlorinated compound with the hydrofluoric acid and the collection of a stream of crude products;
- the separation of the stream of crude products, preferably by distillation, making it possible to recover, on the one hand, a stream of fluorinated compound and, on the other hand, a gas stream mainly comprising hydrochloric acid and also minor amounts of hydrofluoric acid and of fluorinated/oxygenated compounds;
- the treatment of the gas stream comprising hydrochloric acid, hydrofluoric acid and fluorinated/oxygenated compounds according to one of Claims 1 to 9.

11. Process according to Claim 10, in which the catalytic reaction is carried out in the presence of oxygen.

12. Process according to Claim 10 or 11, in which:
- the chlorinated compound is a chlorocarbon, a hydrochlorocarbon, a chlorofluorocarbon, a hydrochlorofluorocarbon, a chloroolefin, a hydrochloroolefin, a chlorofluoroolefin or a hydrochlorofluoroolefin and in which the fluorinated compound is a fluorocarbon, a hydrofluorocarbon, a chlorofluorocarbon, a hydrochlorofluorocarbon, a fluoroolefin, a hydrofluoroolefin, a chlorofluoroolefin or a hydrochlorofluoroolefin;
- preferably, the chlorinated compound is chosen from 1,1,2-trichloroethane, 1,1,1,2,3-pentachloropropane, 1,1,1,3,3-pentachloropropane, 1,1,2,2,3-pentachloropropane, 2,3-dichloro-1,1,1-trifluoropropane, perchloroethylene, 1,2-dichloroethylene, 1,1,2,3-tetrachloropropene, 2,3,3,3-tetrachloropropene, 1,1,3,3-tetrachloropropene, 1,3,3,3-tetrachloropropene, 2-chloro-3,3,3-trifluoropropene, 1-chloro-3,3,3-trifluoropropene and their mixtures; and
- preferably, the fluorinated compound is chosen from pentafluoroethane, 1-chloro-2,2-difluoroethane, 1,3,3,3-tetrafluoropropene, 2,3,3,3-tetrafluoropropene, 2-chloro-3,3,3-trifluoropropene, 1-chloro-3,3,3-trifluoropropene and their mixtures.

13. Plant for the treatment of a gas stream comprising hydrochloric acid, hydrofluoric acid and fluorinated/oxygenated compounds, comprising:
- a pipe for introducing a gas stream comprising hydrochloric acid, hydrofluoric acid and fluorinated/oxygenated compounds (1), feeding a catalytic hydrolysis unit (2);
- a washing unit (4), fed on the one hand by a pipe for collecting the hydrolyzed gas stream (3) resulting from the catalytic hydrolysis unit (2) and on the other hand by a pipe for introducing an acid solution (6);
- a unit for adsorption of impurities (7) comprising a bed of active charcoal, which unit is fed by a pipe for collecting the washed gas stream (5) resulting from the washing unit ;
- a unit for adiabatic or isothermal absorption (9), fed on the one hand by a pipe for collecting the purified stream (8) resulting from the adsorption unit and on the other hand by a pipe for introducing an aqueous solution (10);
- a pipe for collecting a hydrochloric acid solution (12) at the outlet of the adiabatic or isothermal absorption unit (9).

14. Plant according to Claim 13, in which the catalytic hydrolysis unit (2) comprises a bed of active charcoal.

15. Plant according to Claim 13 or 14, in which the pipe for introducing an acid solution (6) does or does not result directly from the pipe for collecting a hydrochloric acid solution (12).

16. Plant according to one of Claims 13 to 15, additionally comprising:
- an additional adsorption unit (13) comprising a silica gel, which unit is fed by the pipe for collecting a hydrochloric acid solution (12); and
- a pipe for collecting a purified hydrochloric acid solution (14) resulting from the additional adsorption unit (13).

17. Plant according to one of Claims 13 to 16, comprising a contribution of boric acid solution to the washing unit (4).

18. Plant according to one of Claims 13 to 17, in which the pipe for introducing a gas stream comprising hydrochloric acid, hydrofluoric acid and fluorinated/oxygenated compounds (1) results from a distillation unit, the distillation unit preferably being fed by a pipe for collecting crude products at the outlet of a catalytic reactor.

19. Plant according to Claim 18, in which the catalytic reactor is fed by a pipe for introducing a chlorinated compound and a pipe for introducing hydrofluoric acid, and the pipe for collecting crude products transports a stream comprising a fluorinated compound, and in which preferably:
- the chlorinated compound is a chlorocarbon, a hydrochlorocarbon, a chlorofluorocarbon, a hydrochlorofluorocarbon, a chloroolefin, a hydrochloroolefin, a chlorofluoroolefin or a hydrochlorofluoroolefin and in which the fluorinated compound is a fluorocarbon, a hydrofluorocarbon, a chlorofluorocarbon, a hydrochlorofluorocarbon, a fluoroolefin, a hydrofluoroolefin, a chlorofluoroolefin or a hydrochlorofluoroolefin;
- more particularly, the chlorinated compound is chosen from 1,1,2-trichloroethane, 1,1,1,2,3-pentachloropropane, 1,1,1,3,3-pentachloropropane, 1,1,2,2,3-pentachloropropane, 2,3-dichloro-1,1,1-trifluoropropane, perchloroethylene, 1,2-dichloroethylene, 1,1,2,3-tetrachloropropene, 2,3,3,3-tetrachloropropene, 1,1,3,3-tetrachloropropene, 1,3,3,3-tetrachloropropene, 2-chloro-3,3,3-trifluoropropene, 1-chloro-3,3,3-trifluoropropene and their mixtures; and
- more particularly, the fluorinated compound is chosen from pentafluoroethane, 1-chloro-2,2-difluoroethane, 1,3,3,3-tetrafluoropropene, 2,3,3,3-tetrafluoropropene, 2-chloro-3,3,3-trifluoropropene, 1-chloro-3,3,3-trifluoropropene and their mixtures.

20. Plant according to Claim 18 or 19, comprising a contribution of oxygen to the catalytic reactor.
